# EUROPÄISCHE PATENTSCHRIFT

(11) **EP 0 966 244 B1**
(45) Veröffentlichungstag und Bekanntmachung des Hinweises auf die Patenterteilung: **19.12.2001**
(21) Anmeldenummer: 98907959.5
(22) Anmeldetag: 20.01.1998
(51) Int. Cl.: A61K 7/00, A61K 7/48

(54) **KOSMETISCHE PIT-EMULSIONEN**
COSMETIC PIT EMULSIONS
EMULSIONS COSMETIQUES OBTENUES SELON LE PROCEDE DE LA TEMPERATURE D'INVERSION DE PHASES

(30) Priorität: 29.01.1997 DE 19703087
(43) Veröffentlichungstag der Anmeldung: 29.12.1999
(73) Patentinhaber: Cognis Deutschland GmbH, 40589 Düsseldorf (DE)
(72) Erfinder: BÖTTCHER, Axel, D-41569 Rommerskirchen (DE); HENSEN, Hermann, D-42781 Haan (DE); SEIPEL, Werner, D-40723 Hilden (DE)
(86) Internationale Anmeldenummer: EP9800279
(87) Internationale Veröffentlichungsnummer: WO9832413

(56) Entgegenhaltungen:
- EP-A- 0 345 586
- WO-A-91/16879
- DE-A- 4 421 208

## Beschreibung

### Gebiet der Erfindung

Die Erfindung betrifft kosmetische PIT-Emulsionen, hergestellt nach der Phaseninversionstemperatur-Methode (PIT), enthaltend Wachsester, Triglyceride, Glyceride und nichtionische Tenside sowie deren Verwendung zur Herstellung von Rückfettungsmitteln.

### Stand der Technik

Jeder Reinigungsvorgang führt zu einer partiellen Entfettung von Haut- und Haaflipiden. Bei intensiver Hautwäsche kann es daher zu Austrocknungserscheinungen kommen, bei häufigem Shampoonieren der Haare besteht die Gefahr, daß die Geschmeidigkeit verlorengeht. Moderne kosmetische Zubereitungen enthalten daher häufig rückfettende Stoffe, die diesen Effekten entgegenwirken. So sind hierfür ausder **DE-PS 2024051** beispielsweise C_{12/18}-Fettsäuremono- und -diester von Anlagerungsprodukten von Ethylenoxid an Glycerin bekannt. Gegenstand der deutschen Patentanmeldung **DE-A1 4337041** (Henkel) ist ein Verfahren zur Herstellung von O/W-Emulsionen nach dem PIT-Verfahren, bei dem man (a) Ölkörper, (b) 0,5 bis 30 Gew.-% nichtionische Emulgatoren mit einem HLB-Wert im Bereich 12 bis 18 und (c) 0,1 bis 30 Gew.-% Ester von Polyolen mit 3 bis 6 Kohlenstoffatomen und Fettsäuren einsetzt; zwingend ist hierbei allerdings die Mitverwendung von Deowirkstoffen, Parfümölen oder Lichtschutzfaktoren. Rückfettungsmittel des Stands der Technik weisen jedoch den Nachteil auf, daß sie sich insbesondere bei höheren Lagertemperaturen als nicht stabil erweisen und einen extremen Viskositätsanstieg zeigen. Vielfach wird zudem beobachtet, daß der rückfettende Effekt nach längerer Lagerung ebenfalls nicht mehr nachweisbar ist. Ein weiteres Problem besteht darin, daß der Tensidgehalt vieler kosmetischer Mittel zu einer unerwünschten Solubilisierung der rückfettenden Emulsionen führt, der auch durch den Einsatz von Wachsen nicht begegnet werden kann.

Demzufolge hat die komplexe Aufgabe der Erfindung darin bestanden, neue rückfettende Systeme zur Verfügung zu stellen, die sich gleichzeitig dadurch auszeichnen, daß sie über ausgezeichnete anwendungstechnische, d.h. sensorische Eigenschaften verfügen und eine hohe Lagerstabilität besitzen.

Insbesondere sollten die Mittel auch bei Lagerung bei höheren Temperaturen eine konstante Viskosität aufweisen und stabil bleiben, d.h. sich nicht entmischen. In Gegenwart von Tensiden sollte es ferner weder zu Solubilisierung noch zu Agglomeration kommen.

### Beschreibung der Erfindung

Gegenstand der Erfindung sind kosmetische PIT-Emulsionen, enthaltend
(a) Wachsester,
(b) Triglyceride,
(c) Partialglyceride und
(d) Fettalkoholpolyglycolether.

Überraschenderweise wurde gefunden, daß Mischungen der Komponenten (a) bis (d) das komplexe Anforderungsprofil in nahezu idealer Weise erfüllen, Die Mischungen besitzen ausgezeichnete rückfettende und avivierende Eigenschaften, die auch bei längerer Lagerung nicht verlorengehen. Selbst bei einer Lagerung von 4 Wochen bei 45°C weisen die Emulsionen eine konstante Viskosität auf und erweisen sich gegenüber Entmischung stabil. In Gegenwart von Tensiden wird keine Solubilisierung beobachtet. Die Erfindung schließt dabei die Erkenntnis ein, daß die Herstellung der Emulsionen nach der PIT-Methode für die Wirksamkeit der Mittel einen kritischen Parameter darstellt.

### Wachsester

Unter Wachsestern, die die Komponente (a) bilden, sind Ester von langkettigen Carbonsäuren mit langkettigen Alkoholen zu verstehen, die vorzugsweise der Formel **(I)** folgen,

**R**^{**1**}**CO-OR**^{**2**} **(I)**

in der R¹CO für einen gesättigten und/oder ungesättigten Acylrest mit 6 bis 22, vorzugsweise 12 bis 18 Kohlenstoffatomen und R² für einen Alkyl- und/oder Alkenylrest mit 6 bis 22, vorzugsweise 12 bis 18 Kohlenstoffatomen steht. Typische Beispiele sind Ester von Capronsäure, Caprylsäure, 2-Ethylhexansäure, Caprinsäure, Laurinsäure, isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen mit Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Vorzugsweise werden Cetylpalmitat, Cetylstearat, Cetylisostearat, Cetyloleat, Cetylbehenat, Cetylerucat, Stearylpalmitat, Stearylstearat, Stearylisostearat, Stearyloleat, Stearylbehenat, Stearylerucat, Isosteraylpalmitat, Isostearylstearat, Isostearylisostearat, Isostearyloleat, Isostearylbehenat, Isostearylerucat, Oleylpalmitat, Oleylstearat, Oleylisostearat, Oleyloleat, Oleylbehenat, Oleylerucat, Behenylpalmitat, Behenylstearat, Behenylisostearat, Behenyloleat, Behenylbehenat, Behenyloleat sowie deren Mischungen eingesetzt. Weiterhin können als Wachsester auch Ester der genannten Alkohole mit Fruchtsäuren, also beispielsweise Äpfel- Wein- oder Citronensäure, Fruchtwachse sowie Siliconwachse eingesetzt werden.

### Triglyceride

Unter Triglyceriden, die im Sinne der Erfindung die Komponente (b) bilden, sind Stoffeder Formel **(II)** zu verstehen, 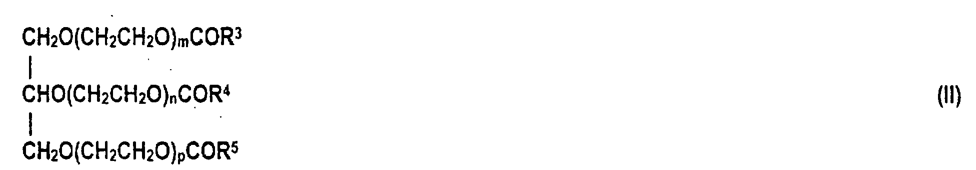 in der R³CO, R⁴CO und R⁵CO unabhängig voneinander für lineare oder verzweigte, gesättigte und/oder ungesättigte; gegebenenfalls hydroxy- und/oder epoxysubstituierte Acylreste mit 6 bis 22, vorzugsweise 12 bis 18 Kohlenstoffatomen und die Summe (m+n+p) für 0 oder Zahlen von 1 bis 100, vorzugsweise 20 bis 80 steht. Die Triglyceride können natürlicher Herkunft sein oder auf synthetischem Wege hergestellt werden. Vorzugsweise handelt es sich um hydroxy- und/oder epoxyfunktionalisierte Stoffe, wie beispielsweise Ricinusöl oder gehärtetes Ricinusöl, epoxidiertes Ricinusöl, Ringöffnungsprodukte von epoxidierten Ricinusölen unterschiedlicher Epoxidzahlen mit Wasser sowie Anlagerungsprodukten von durchschnittlich 1 bis 100, vorzugsweise 20 bis 80 und insbesondere 40 bis 60 Mol an diese genannten Triglyceride.

### Partialglyceride

Als Komponente (c) kommen Partialglyceride, also Monoglyceride,. Diglyceride und deren technische Gemische in Frage, die herstellungsbedingt noch geringe Mengen Triglyceride enthalten können. Die Partialglyceride folgen vorzugsweise der Formel **(III)**,

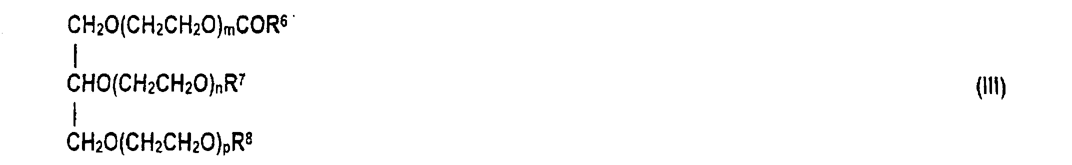

in der R⁶CO für einen linearen oder verzweigten, gesättigten und/oder ungesättigten Acylrest mit 6 bis 22, vorzugsweise 12 bis 18 Kohlenstoffatomen, R⁷ und R⁸ unabhängig voneinander für R⁶CO oder OH und die Summe (m+n+p) für 0 oder Zahlen von 1 bis 100, vorzugsweise 5 bis 25 steht, mit der Maßgabe, daß mindestens einer der beiden Reste R⁷ und R⁸OH bedeutet. Typische Beispiele sind Monound/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure; Linolsäure, Linolensäure, Elaeostearinsäure, Arachinsäure, Gadoleinsäure, Behensäure und Erucasäure sowie deren technische Mischungen. Vorzugsweise werden technische Laurinsäureglyceride, Palmitinsäureglyceride, Stearinsäureglyceride, Isostearinsäureglyceride, Ölsäureglyceride, Behensäureglyceride und/oder Erucasäureglyceride eingesetzt, welche einen Monoglyceridanteil im Bereich von 50 bis 95, vorzugsweise 60 bis 90 Gew.-% aufweisen.

### Fettalkoholpolyglycolether

Als Komponente (d) kommen schließlich Fettalkoholpolyglycolether der Formel **(IV)** in Betracht,

**R**^{**9**}**O(CH**_{**2**}**CH**_{**2**}**O)**_{**q**}**H (IV)**

in der R⁹ für einen linearen oder verzweigten Alkyl- und/oder Alkenylrest mit 6 bis 22 Kohlenstoffatomen und q für Zahlen von 1 bis 50 steht, Typische Beispiele sind Anlagerungsprodukte von durchschnittliche 1 bis 50, vorzugsweise 5 bis 25 an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylatkohol, Eiaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Tenside können dabei sowohl eine konventionell breite als auch eine eingeengte Homologenverteilung aufweisen. Besonders bevorzugt sind Anlagerungsprodukte von durchschnittlich 10 bzw. 20 Mol Ethylenoxid an Cetearylalkohol, Stearylalkohol und/ oder Behenylalkohol.

### Gewerbliche Anwendbarkeit

In einer bevorzugten Ausführungsform der Erfindung werden kosmetische PIT-Emulsionen beansprucht, die die Komponenten (a) bis (d) in den folgenden Mengen enthalten:
(a) 25 bis 50, vorzugsweise 30 bis 40 Gew.-% Wachsester,
(b) 1 bis 10, vorzugsweise 2 bis 8 Gew.-% Triglyceride,
(c) 1 bis 10, vorzugsweise 2 bis 8 Gew.-% Partialglyceride und
(d) 1 bis 20, vorzugsweise 10 bis 15 Gew.-% Fettalkoholpolyglycolether,
mit der Maßgabe, daß sich die Mengenangaben mit Wasser und gegebenenfalls weiteren üblichen Hilfs- und Zusatzstoffen zu 100 Gew.-% ergänzen. Ein weiterer Gegenstand der Erfindung betrifft die Verwendung dieser PIT-Emulsionen zur Herstellung von Rückfettungsmitteln.

### Kosmetische Zubereitungen

Die erfindungsgemäßen Zubereitungen, wie beispielsweise Haarshampoos, Haarlotionen, Schaumbäder, Cremes, Lotionen oder Salben, können ferner als weitere Hilfs- und Zusatzstoffe milde Tenside, Ölkörper, Co-Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fett-alkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkohol-carbonate, Guerbetcarbonate, Ester der Benzoesäure mit linearen und/oder verzweigten C₆-C₂₂-Alko-holen (z.B. Finsolv® TN), Dialkylether, Siliconöle und/oder aliphatische bzw. naphthenische Kohlen-wasserstoffe in Betracht.

Als **Co-Emulgatoren** kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(3) Alkylmono- und -oligoglycoside mit 8 bis 22 Kohlenstoffatomen im Alkylrest und deren ethoxylierte Analoga;
(4) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(5) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylgiucoside (z.B. Methylglucosid, Butylglucosid, Laurylglucosid) sowie Polyglucoside (z.B. Cellulose);
(6) Trialkylphosphate sowie Mono-, Di- und/oder Tri-PEG-alkylphosphate;
(7) Wollwachsalkohole;
(8) Polysiloxan-Polyalkyl-Polyether-Copolymere bzw. entsprechende Derivate;
(9) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20** **36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Suifonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter biogenen Wirkstoffen sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkyl-modifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyt-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoyimethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die. gesamte Mischung, eingesetzt. Der Gesamtanteil der Hilfs- und Zusatzstoffe kann 1 bis 50, vorzugsweise 5 bis 40 Gew.-% - bezogen auf die Mittel - betragen.

### Beispiele

Rückfettungsmittel unterschiedlicher Zusammensetzung wurden auf ihre Performance untersucht. Die Viskosität der Zubereitungen wurde nach der Brookfield-Methode in einem RVF-Viskosimeter (Spindel 1,10 Upm) einmal unmittelbar nach der Herstellung (20°C) und ein weiteres Mal nach einer Lagerzeit von 4 Wochen bei 45°C gemessen. Die Stabilität der Formulierungen wurde nach Lagerung (4 w, 45°C) optisch bestimmt. Dabei bedeutet "+" stabil und "-" Phasentrennung. Die rückfettende Wirkung wurde durch ein Panel geschulter Probanden subjektiv beurteilt. Dabei bedeutet 1 = sehr gut und 3 = durchschnittlich. Angegeben ist der Mittelwert von 5 Messungen für ein Mittel unmittelbar nach dessen Herstellung und für das gleiche Mittel nach Lagerung (4 w, 45°C). Die Ergebnisse sind in Tabelle 1 zusammengefaßt. Die Rezepturen R1 und R2 sind erfindungsgemäß, die Rezepturen R3 bis R6 dienen zum Vergleich.

**Tabelle 1:**

| Performance von Rückfettungsmitteln (Mengenangaben als Gew.-%) | | | | | | |
|---|---|---|---|---|---|---|
| **Zusammensetzung / Performance** | **R1** | **R2** | **R3** | **R4** | **R5** | **R6** |
| Cetyl Palmitate | 30 | 40 | 30 | 30 | 30 | - |
| Hydrogenated Castor Oil | 4 | 6 | - | 4 | 4 | 4 |
| Glyceryl Stearate | 2 | 3 | 6 | - | 10 | 30 |
| Beheneth-10 | 8 | 12 | 8 | 10 | - | 10 |
| Wasser | ad 100 | | | | | |
| ***Viskosität - sofort [mPas]*** | 6.000 | 6.400 | 6.000 | 5.800 | 6.200 | 6.000 |
| ***Viskosität - nach Lagerung [mPas]*** | 6.100 | 6.400 | 12.000 | 17.000 | 15.000 | 12.000 |
| ***Stabilität*** | + | + | - | - | - | - |
| ***Rückfettungseffekt - sofort*** | 1,0 | 1,5 | 1,5 | 2,0 | 3,0 | 3,0 |
| ***Rückfettungseffekt - nach Lagerung*** | 1,0 | 1,5 | 3,0 | 3,0 | 3,0 | 3,0 |

## Patentansprüche

1. Kosmetische PIT-Emulsionen, enthaltend
(a) Wachsester,
(b) Triglyceride,
(c) Partialglyceride und
(d) Fettalkoholpolyglycolether.

2. Kosmetische PIT-Emulsionen nach Anspruch 1, **dadurch gekennzeichnet, daß** man Wachsester der Formel (I) einsetzt,
**R**^{**1**}**CO-OR**^{**2**} **(I)**
in der R¹CO für einen gesättigten und/oder ungesättigten Acylrest mit 6 bis 22 Kohlenstoffatomen und R² für einen Alkyl- und/oder Alkenylrest mit 6 bis 22 Kohlenstoffatomen steht.

3. Kosmetische PIT-Emulsionen nach den Ansprüchen 1 und 2, **dadurch gekennzeichnet, daß** man Triglyceride der Formel **(II)** einsetzt, 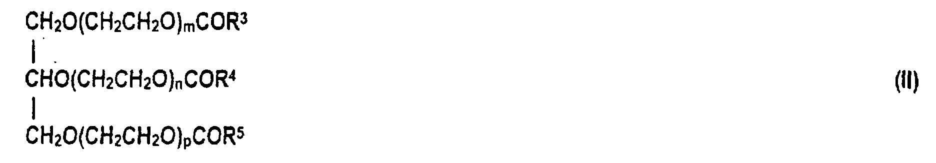 in der R³CO, R⁴CO und R⁵CO unabhängig voneinander für lineare oder verzweigte, gesättigte und/oder ungesättigte, gegebenenfalls hydroxy- und/oder epoxysubstituierte Acylreste mit 6 bis 22 Kohienstoffatomen und die Summe (m+n+p) für 0 oder Zahlen von 1 bis 100 steht.

4. Kosmetische PIT-Emulsionen nach den Ansprüchen 1 bis 3, **dadurch gekennzeichnet, daß** man Partialglyceride der Formel **(III)** einsetzt, 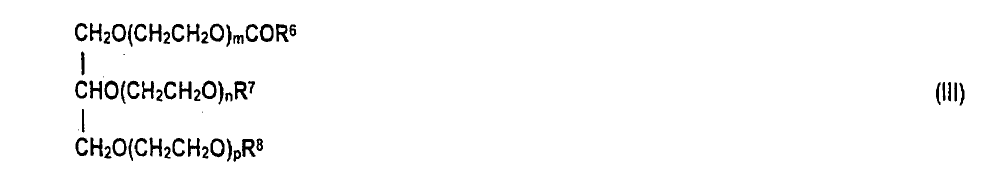 in der R⁶CO für einen linearen oder verzweigten, gesättigten und/oder ungesättigten Acylrest mit 6 bis 22 Kohienstoffatomen, R⁷ und R⁸ unabhängig voneinander für R⁶CO oder OH und die Summe (m+n+p) für 0 oder Zahlen von 1 bis 100 steht, mit der Maßgabe, daß mindestens einer der beiden Reste R⁷ und R⁸ OH bedeutet.

5. Kosmetische PIT-Emulsionen nach den Ansprüchen 1 bis 4, **dadurch gekennzeichnet, daß** man Fettalkoholpolyglycolether der Formel **(IV)** einsetzt,
**R**^{**9**}**O(CH**_{**2**}**CH**_{**2**}**O)**_{**q**}**H (IV)**
in der R⁹ für einen linearen oder verzweigten Alkyl- und/oder Alkenylrest mit 6 bis 22 Kohlenstoffatomen und q für Zahlen von 1 bis 50 steht.

6. Kosmetische PIT-Emulsionen nach den Ansprüchen 1 bis 5, **dadurch gekennzeichnet, daß** sie
(a) 25 bis 50 Gew.-% Wachsester,
(b) 1 bis 10 Gew.-% Triglyceride,
(c) 1 bis 10 Gew.-% Partialglyceride und
(d) 1 bis 20 Gew.-% Fettalkoholpolyglycolether
enthalten, mit der Maßgabe, daß sich die Mengenangaben mit Wasser und gegebenenfalls weiteren üblichen Hilfs- und Zusatzstoffen zu 100 Gew.-% ergänzen.

7. Verwendung von PIT-Emulsionen nach Anspruch 1 zur Herstellung von Rückfettungsmitteln.

## Claims

1. Cosmetic PIT emulsions containing
(a) wax esters,
(b) triglycerides,
(c) partial glycerides and
(d) fatty alcohol polyglycol ethers.

2. Cosmetic PIT emulsions as claimed in claim 1, **characterized in that** wax esters corresponding to formula **(I)**:
**R**^{**1**}**CO-OR**^{**2**} **(I)**
in which R¹CO is a saturated and/or unsaturated acyl group containing 6 to 22 carbon atoms and R² is an alkyl and/or alkenyl group containing 6 to 22 carbon atoms, are used.

3. Cosmetic PIT emulsions as claimed in claims 1 and 2, **characterized in that** triglycerides corresponding to formula **(II)**: 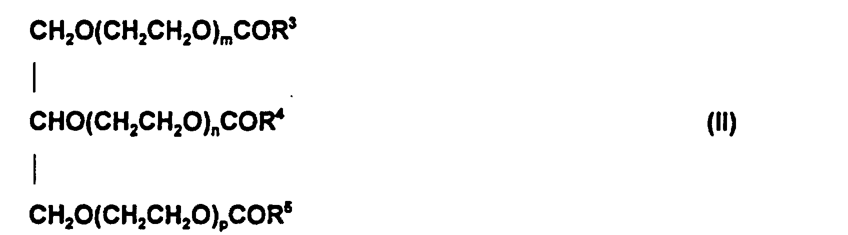 in which R³CO, R⁴CO and R⁵CO independently of one another represent linear or branched, saturated and/or unsaturated, optionally hydroxy- and/or epoxy-substituted acyl groups containing 6 to 22 carbon atoms and the sum (m+n+p) is 0 or a number of 1 to 100, are used.

4. Cosmetic PIT emulsions as claimed in claims 1 to 3, **characterized in that** partial glycerides corresponding to formula **(III)**: 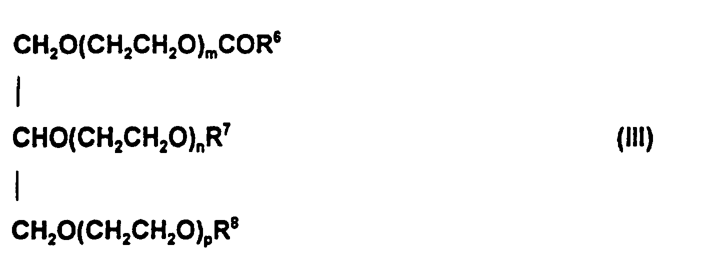 in which R⁶CO is a linear or branched, saturated and/or unsaturated acyl group containing 6 to 22 carbon atoms, R⁷ and R⁸ independently of one another have the same meaning as R⁶CO or represent OH and the sum (m+n+p) is O or to a number of 1 to 100, with the proviso that at least one of the two substituents R⁷ and R⁸ represents OH,
are used.

5. Cosmetic PIT emulsions as claimed in claims 1 to 4, **characterized in that** fatty alcohol polyglycol ethers corresponding to formula **(IV)**:
**R**^{**9**}**O(CH**_{**2**}**H**_{**2**}**O)**_{**q**}**H (IV)**
in which R⁹ is a linear or branched alkyl and/or alkenyl group containing 6 to 22 carbon atoms and q is a number of 1 to 50, are used.

6. Cosmetic PIT emulsions as claimed in claims 1 to 5, **characterized in that** they contain
(a) 25 to 50% by weight of wax esters,
(b) 1 to 10% by weight of triglycerides,
(c) 1 to 10% by weight of partial glycerides and
(d) 1 to 20% by weight of fatty alcohol polyglycol ethers, with the proviso that the quantities shown add up to 100% by weight
with water and optionally other typical auxiliaries and additives.

7. The use of the PIT emulsions claimed in claim 1 for the production of refatting systems.

## Revendications

1. Emulsions PIT cosmétiques contenant :
a) des esters de cire
b) des triglycérides
c) des glycérides partiels et
d) des esters de polyglycol et d'alcool gras.

2. Emulsions PIT cosmétiques selon la revendication 1,
**caractérisées en ce qu'**
on met en oeuvre des esters de cire de formule (I)
R¹CO-OR² (I)
dans laquelle R¹CO représente un reste acyle saturé et/ou non saturé ayant de 6 à 22 atomes de carbone et R² représente un reste alkyle et/ou alkényle ayant de 6 à 22 atomes de carbone.

3. Emulsions PIT cosmétiques selon les revendications 1 et 2,
**caractérisées en ce qu'**
on met en oeuvre des triglycérides de formule (II) 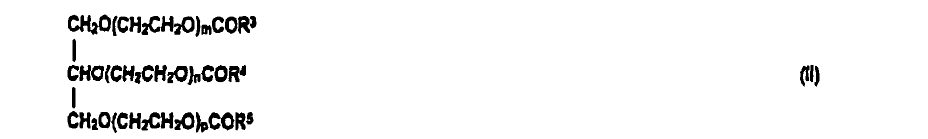 dans laquelle R3CO, R4CO, R5CO indépendamment les uns des autres, représentent des restes acyle linéaires ou ramifiés, saturés et/ou non saturés éventuellement substitués par un hydroxy et/ou par un époxyde ayant de 6 à 22 atomes de carbone et la somme (m+n+p) représente O ou des nombres allant de 1 à 100.

4. Emulsions PIT cosmetiques selon les revendications 1 à 3,
**caractérisées en ce qu'**
on met en oeuvre des glycérides partiels de formule (III) 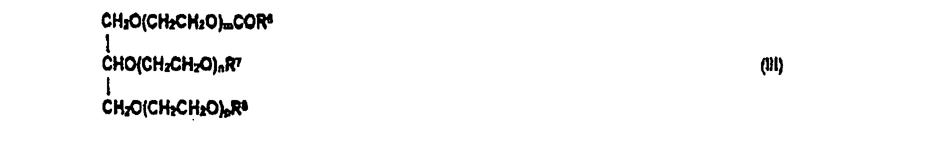 dans laquelle R⁶CO représente un reste acyle, linéaire ou ramifié, saturé et/ou non saturé ayant de 6 à 22 atomes de carbone, R7 et R8 indépendamment l'un de l'autre représentent R⁶CO ou OH et la somme (m+n+p) représente O ou des nombres allant de 1 à 100 avec la précision qu'au moins un des deux restes R⁷ et R⁸ signifie OH.

5. Emulsions PIT cosmétiques selon les revendications 1 à 4,
**caractérisées en ce qu'**
on met en oeuvre des ethers d'alcool gras et de polyglycol de formule (IV)
R⁹O(CH₂CH₂O)_{q}H (IV)
dans laquelle R⁹ représente un reste alkyle et/ou alkényle linéaire ou ramifié, ayant de 6 à 22 atomes de carbone et q représente des nombres allant de 1 à 50.

6. Emulsions PIT cosmétiques selon les revendications 1 à 5,
**caractérisées en ce qu'**
elles contiennent
a) de 25 à 50 % en poids d'esters de cire
b) de 1 à 10 % en poids de triglycérides
c) de 1 à 10 % en poids de triglycérides partiels, et
d) de 1 à 20 % en poids d'éther d'alcool gras et de polyglycol,
avec la précision que les données de quantités se complétent avec l'eau et éventuellement avec d'autres adjuvants et additifs usuels, à 100 % en poids.

7. Utilisation des émulsions PIT selon la revendication 1,
pour l'obtention d'agents de regraissage.
